# EUROPÄISCHE PATENTSCHRIFT

(11) **EP 2 758 063 B1**
(45) Veröffentlichungstag und Bekanntmachung des Hinweises auf die Patenterteilung: **30.12.2015**
(21) Anmeldenummer: 12780404.5
(22) Anmeldetag: 20.09.2012
(51) Int. Cl.: A61K 33/06, A61P 39/00, A61P 29/02, A61P 39/04, A61P 43/00

(54) **KLINOPTILOLITH ZUR VERWENDUNG BEI DER AUSLEITUNG VON CAESIUMIONEN**
KLINOPTILOLITH FOR USE IN THE REMOVAL OF CAESIUM IONS
CLINOPTILOLITHE DESTINÉ A ÊTRE UTILISÉ POUR LE RETRAIT DES IONS DE CAESIUM

(30) Priorität: 23.09.2011 DE 102011053908
(43) Veröffentlichungstag der Anmeldung: 30.07.2014
(73) Patentinhaber: Froximun AG, 38838 Schlanstedt (DE)
(72) Erfinder: STEIMECKE, Günter, 38855 Wernigerode (DE); GÖRNER, Thomas, 38838 Schlanstedt (DE)
(74) Vertreter: Sperling, Thomas
(86) Internationale Anmeldenummer: PCT/DE2012/100290
(87) Internationale Veröffentlichungsnummer: WO 2013/041090

(56) Entgegenhaltungen:
- F. A. MUMPTON: "Colloquium Paper: La roca magica: Uses of natural zeolites in agriculture and industry", PROCEEDINGS OF THE NATIONAL ACADEMY OF SCIENCES, Bd. 96, Nr. 7, 30. März 1999 (1999-03-30), Seiten 3463-3470, XP055045116, ISSN: 0027-8424, DOI: 10.1073/pnas.96.7.3463
- PHILLIPPO, M; GVOZDANOVIC, S, GVOZDANOVIC, D; CHESTERS, JK; PATERSON, E; MILLS, CF: "Reduction of radiocaesium absorbtion by sheep consuming feed contaminated with fallout from Chernobyl", THE VETERINARY RECORD, Bd. 122, Nr. 23, 4. Juni 1988 (1988-06-04) , Seiten 560-563, XP9164985, ISSN: 0042-4900
- JANDL, J; NOVOSAD, J: "In vivo reduction of radiocaesium by modified cliniptilolite in sheep", VETERINARNI MEDICINA (PRAGUE), Bd. 40, Nr. 8, 20. Januar 1995 (1995-01-20), Seiten 237-241, XP009164995,
- Schulze, N; Hense, H-J: "Natürliches Zeolith (Klinoptilolith) im Gesundheitswesen - Forschung und Praxis", Zeolith-Welt , 1. Januar 2011 (2011-01-01), XP002687684, Gefunden im Internet: URL:http://www.zeolithwelt.de/news/zeolith -gesundheit-medizin [gefunden am 2012-11-21]
- Karl Hecht: "Natur-Klinoptilolith-Zeolith gegen Atomreaktorstrahlung", , 1. Januar 2011 (2011-01-01), XP055045130, Gefunden im Internet: URL:http://www.zeolith-bentonit-versand.de /download/Prof. Dr. Karl Hecht-Atomreaktorstrahlung.pdf [gefunden am 2012-11-21]
- DATABASE WPI Week 199713 Thomson Scientific, London, GB; AN 1997-143696 XP002687685, -& RU 2 063 229 C1 (A MED SIBE BIOCHEM INST) 10. Juli 1996 (1996-07-10)
- ABDELRAHIM ABUSAFA ET AL: "Removal of 137Cs from aqueous solutions using different cationic forms of a natural zeolite: clinoptilolite", SEPARATION AND PURIFICATION TECHNOLOGY, Bd. 28, Nr. 2, 1. August 2002 (2002-08-01) , Seiten 103-116, XP055045112, ISSN: 1383-5866, DOI: 10.1016/S1383-5866(02)00042-4

## Beschreibung

Die Erfindung betrifft die Verwendung von Klinoptilolith oder klinoptilolithhaltigen Gemischen zur Herstellung von Medizinprodukten oder anderen pharmazeutischen Produkten, zur Aufnahme und Ausleitung von Caesiumionen aus dem menschlichen oder tierischen Organismus. Insbesondere ist ein Medizinprodukt mit dem modifizierten Klinoptilolith geeignet, sowohl die Konzentration der Aufnahme von radioaktiven Caesiumionen in den menschlichen oder tierischen Körper als auch die Konzentration bereits im menschlichen oder tierischen Körper vorhandener und resorbierter radioaktiver Caesiumionen zu reduzieren.

Caesium ist ein Metall aus der Gruppe der Alkalimetalle, welches in der Natur ausschließlich in Form seiner Salze bzw. seiner chemischen Verbindungen auftritt. Caesium ist in seinen Verbindungen üblicherweise ein einfach positiv geladenes Kation. Als chemisches Element wurde Caesium im Jahre 1861 durch Kirchhoff und Bunsen auf spektrosköpischem Wege im Mineralwasser von Bad Dürkheim entdeckt. Es tritt meist mit anderen Alkalimetallsalzen vergesellschaftet beispielsweise in Salzlagerstätten oder im Meerwasser auf und ist auch in einigen seltenen Mineralien zu finden. Seine Salze sind nicht toxisch und werden im menschlichen Körper wie das Element Kalium verstoffwechselt. Über eine mögliche biologische Bedeutung des Elements Caesium innerhalb der Ernährung, beispielsweise als Spurenelement, ist nichts bekannt.

Das Element Caesium ist natürlich vorkommend ein sogenanntes Reinelement, das heißt, es besteht ausschließlich aus dem stabilen und nichtradioaktiven Isotop Caesium-133 bzw. ¹³³Cs. Seit Nutzung der Kernspaltung von Uran-235 nach dem Jahre 1945 sowohl für militärische als auch für zivile Zwecke, zum Beispiel für die Energiegewinnung, fällt ein bedeutendes Spalt- bzw. Abfallprodukt, das Caesium-137, an. Caesium-137 entsteht neben anderen Caesiumisotopen und weiteren Spaltprodukten bei einem Neutronenbeschuss von Uran-235 und dessen auf diesem Wege induzierter Kernspaltung im Kernreaktor. Caesium-137 ist radioaktiv, wobei es mit einer Halbwertzeit von 30,17 Jahren unter Aussendung von Gammastrahlung seinerseits in das stabile Bariumisotop-137 zerfällt.

Der Umgang mit verbrauchten Brennstäben und gespaltenem Material innerhalb der Kernkraftwerke ist stets verbunden mit einer denkbaren Freisetzung von Caesium-137. Die Wahrscheinlichkeit dafür ist jedoch sehr gering.

Größere Mengen an Caesium-137 gelangten durch oberirdische Kernwaffenversuche und durch die Reaktorunfälle sowohl in Tschernobyl als auch von Fukushima in die Umwelt. Große Gebiete in Europa und auch von Deutschland sind nach dem Reaktorunfall in Tschernobyl durch einen Fallout an Caesium-137 mehr oder weniger stark belastet worden. Lebewesen sind nicht in der Lage, zwischen dem harmlosen Isotop Caesium-133 und dem sehr gefährlichen Isotop Caesium-137 zu unterscheiden. Beide Isotope verhalten sich chemisch völlig gleich. Da, wie bereits erwähnt, auch das radioaktive Caesium-137 im menschlichen und tierischen Körper wie das Element Kalium aufgenommen und verstoffwechselt wird, strahlt das Isotop Caesium-137 natürlich auch innerhalb der Zellen in den Organismus weiter. Die dabei freigesetzte starke Gammastrahlung ionisiert die unmittelbare Umgebung und schädigt auf diese Weise die Zellen der Lebewesen irreversibel. Es kommt in Abhängigkeit von der einwirkenden Dosis und der Zeitdauer der Belastung zu Strahlenschäden oder gar zur Strahlenkrankheit. Die Inkorporation von Caesium-137 hat auf die Gesundheit der Lebewesen eine ganz besonders negative Wirkung zur Folge.

Caesium-137 wird besonders durch verschiedene Pilze in Waldgebieten angereichert. Für wildlebende Tiere dienen Pilze unter anderem als Nahrungsquelle. In verschiedenen Gebieten, insbesondere in Süddeutschland, ist die messbare Belastung durch das Caesium-137 immer noch derart hoch, dass Wildbret vor einem möglichen Verzehr verbindlich auf eine Belastung mit Caesium-137 geprüft werden muss. Liegt der Wert zu hoch, muss das Tier entsorgt werden. Bezüglich des Isotops Caesium-137 ist bis zum heutigen Tage kein Mittel zum Schutz vor einer denkbaren oder möglichen Kontamination durch seine unfreiwillige Aufnahme in den menschlichen Körper verfügbar.

Bisher ist in diesem Zusammenhang lediglich eine als nennenswert zu bezeichnende präventive Maßnahme die Gabe von Kaliumiodid-Tabletten anzuführen, die verhindern soll, dass im Falle einer nuklearen Katastrophe oder einer ungewollten Freisetzung von Radioaktivität entstehendes radioaktives lod von der Schilddrüse aufgenommen werden kann. Die Schilddrüse wird sozusagen vor einer möglichen Kontamination mit radioaktivem Jod durch das nichtradioaktive lod gesättigt. Die Einnahme der Kaliumiodid-Tabletten muss aus diesem Grunde unbedingt vor der möglichen Kontamination geschehen.

Aus Proc. Natl. Acad. Sci. USA 1999, 96(7), S. 3463 bis 3470**,** ist die Anwendung von natürlichen Zeolithen in der Landwirtschaft und in der Industrie bekannt. Insbesondere wird die Nutzung von Klinoptilolith bei der Verringerung von Caesium-137 nach radioaktivem Niederschlag infolge von Nukleartests und Unfällen in Böden beschrieben. Dabei wird erwähnt, dass in Bulgarien Zeolithpillen und Zeolithgebäck für den menschlichen Konsum hergestellt wurden, um der Belastung mit Caesium-137 und Strontium-90 infolge des Tschernobyl-Unfalls entgegenzuwirken. Dabei tauscht das Zeolith Caesium-137 und Strontium-90 im Magen-Darm-Trakt aus, so dass beides auf normalem Wege ausgeschieden werden kann. Somit soll die Aufnahme in den Körper minimiert werden.

In Vet. Record, 1988, 122(23), S. 560-563**,** sind Versuche zur Verringerung der Radiocaesiumabsorption bei Schafen beschrieben, die radioaktiv kontaminiertes Futter infolge der Reaktorkatastrophe von Tschernobyl konsumiert hatten. Dabei wurde unter anderem Klinoptilolith als Caesium-Absorber bestimmt, wobei 90 Prozent der Partikel des Klinoptiloliths größer als 250 Mikrometer waren. Wenn Caesium *in vivo* angewendet wird, reduziert sich die Absorption von Caesium, welche infolge der Konsumierung von kontaminiertem Futter auftritt. Das Klinoptilolithmaterial ändert nicht die Ausscheidungsrate von vorabsorbiertem Caesium, welche seit der Zeit der Beendigung der Kontamination variierte.

Die Druckschrift Veterinarni Medicina (Prague), 1995, 40(8), 237 bis 241**,** beschreibt die Verringerung von Radiocaesium durch modifiziertes Klinoptilolithmaterial bei *in vivo*-Versuchen in Schafen. Die Modifikation des Klinoptiloliths besteht in einer Immobilisierung von Eisen-(II)-Hexacyanoferrat auf Klinoptilolith. Dabei wurde herausgefunden, dass die Anwendung eines auf diese Weise modifizierten Klinoptiloliths sowohl die primäre als auch die sekundäre Resorption von Caesium-137 durch Unterbrechung des enteralen Zyklus beeinflusst. So zeigte sich, dass das modifizierte Klinoptilolith beide Male die Ausscheidung von Caesium-137 aus dem Schafkörper beschleunigt. Der ganze Effekt im Falle der konstanten Aufnahme von kontaminiertem Futter und der gleichzeitigen täglichen Einnahme von 50 Gramm des modifizierten Klinoptiloliths führte zu einer fünfzehn- bis fünfzigfachen Verringerung der Gleichgewichtskonzentration. Die Wirkung des modifizierten Klinoptiloliths wurde schließlich mit der Wirkung von nicht-modifiziertem Klinoptilolith verglichen. Dabei wurde bei nicht-modifiziertem Klinoptilolith eine zehnfach geringere Sorptionswirkung festgestellt.

Nach Schulze, N., Hense, H.-J.: "Natürliches Zeollith (Klinoptilolith) im Gesundheitswesen - Forschung und Praxis", Zeolith-Welt, 1. Januar 2011 (2011-01-01), URL: http://www.zeolithwelt.de/news/zeolith-gesundheit-medizin**,** ist Klinoptilolith dank seiner Eigenschaft als Molekularsieb in der Lage, unter anderem Caesium-137 im Körper von Lebewesen zu binden und herauszufiltern. Des Weiteren ist beschrieben, dass die Beimischung von Klinoptilolith in Nahrungsmitteln Untersuchungen zufolge zum Beispiel einen drei- bis fünffach stärkeren Abbau von Caesium-137 im menschlichen Körper bewirkt. Zeolith selbst wird nicht verdaut oder gespeichert. Der menschliche Körper scheidet das Mineral nach Aufnahme der giftigen Radionuklide aus.

Karl Hecht: "Natur-Klinoptilolith-Zeolith gegen Atomreaktorstrahlung", 1. Januar 2011 (2011-01-01), URL: http://www.zeolith-bentonit-versand.de/download/Prof. Dr. Karl Hecht-Atomreaktorstrahlung.pdf**,** berichtet, dass bei Hühnern Caesium-137 aus allen untersuchten Organen mit Hilfe von Klinoptilolithbeimengungen von 2,5 bis 10 Prozent Anteil am Gesamtfutter eliminiert werden konnte. Auch sei die dekontaminative Wirkung bei präventiver Applikation an danach bestrahlten braunen Ratten beobachtet worden. Des Weiteren sei Klinoptilolith bereits in Tschernobyl zum Einsatz gekommen, wobei nach Angabe des Autors einem Teil der Opfer geholfen werden konnte. Klinoptilolith erwies sich nach Aussage des Autors besonders dann als ein nützliches Mittel zum vorbeugenden Schutz gegen Strahlungen, wenn es mit anderen Naturmitteln, zum Beispiel mit Vitaminen, appliziert wurde. Insbesondere die Kombination mit den Vitaminkomplexen A, C, E oder A, B, C zeigte sich als Erfolg versprechend. Bei der Ausleitung von radioaktivem Caesium-137 erwies sich besonders der Komplex mit Blaualgen als effektiv.

Aus der RU 2 063 229 C1 ist die Anwendung von zeolithhaltigem Tuffstein im Bereich der Radiomedizin, genauer gesagt, zur Behandlung bei einer radioaktiven Kontamination im menschlichen Körper, bekannt.

Zum Schutz der Bevölkerung vor Caesium-137 nach dessen Freisetzung und zur Dekontamination von Personen, die bereits mit Caesium-137 kontaminiert worden sind, ist die Entwicklung eines geeigneten, kostengünstigen Mittels notwendig, mit dem die stark strahlenschädigende Wirkung des Caesium-137-Isotops effizient reduziert oder begrenzt werden kann. Die Aufgabe der Erfindung besteht darin, ein derartiges Mittel bereitzustellen.

Die Lösung der Aufgabe der Erfindung besteht in Klinoptilolith oder klinoptilolithhaltigen Gemischen zur Verwendung als Medizinprodukt oder eines anderen pharmazeutischen Produktes, zur Aufnahme und Ausleitung von Caesiumionen aus dem menschlichen oder tierischen Körper. Erfindungsgemäß ist das Klinoptilolith durch Reinigung und Zerkleinerung modifiziert, dadurch gekennzeichnet, dass die Partikel des modifizierten Klinoptiloliths nach der Zerkleinerung Partikelgrößen von 2 bie 15 µm aufweisen. Durch die Modifizierung und Aktivierung des Klinoptiloliths weisen dessen Partikel vorteilhaft Zetapotenziale von 20 bis 26 mV und vorzugsweise eine spezifische Oberfläche von 30 bis 60 m²/g auf.

Es wurde überraschend gefunden, dass modifiziertes Klinoptilolith oder entsprechende klinoptilolithhaltige Gemische, insbesondere das Medizinprodukt "froximun toxaprevent® pure", die Eigenschaft besitzen, sowohl *in vitro* als auch *in vivo* Caesiumionen zu binden und via Magen-Darm-Trakt aus dem Körper über den Zeitraum der Einnahme auszuscheiden. Die Selektivität gegenüber Caesiumionen ist bei diesem Vorgang sehr groß. Wird ein solches klinoptilolithhaltiges Präparat bzw. Medizinprodukt oder pharmazeutisches Produkt gemeinsam mit der Nahrung verabreicht, so wird in Abhängigkeit von der Dosierung und der vorliegenden Caesiumkonzentrationen die Aufnahme von Caesiumverbindungen bzw. von Caesiumsalzen in den menschlichen und tierischen Körper reduziert.

Die sehr gute Bindung von Caesium an das modifizierte Klinoptilolith ist sehr überraschend, wobei man dem erfindungsgemäßen Ziel zum Beispiel mit Hilfe des bereits genannten, in Deutschland zugelassenen Medizinproduktes "froximun toxaprevent® pure" gerecht werden kann. Das bereits in der DE 10 2005 053 090 A1 und WO 2010/057849 A1 beschriebene Wirkungsspektrum gegenüber den dort näher bezeichneten Schadstoffen kann um Caesium erweitert werden. Ein Vorhandensein von radioaktivem Caesium-137 nach möglicher Freisetzung von Radioaktivität kann durch die Einnahme eines entsprechenden Medizinproduktes oder anderen pharmazeutischen Produktes im Organismus in seiner Konzentration reduziert werden.

Vorteilhafterweise ist das verwendete Medizinprodukt oder andere pharmazeutische Produkt mit dem modifizierten Klinoptilolith geeignet, bei vorbeugender und/oder rechtzeitiger Einnahme sowohl die Aufnahme von radioaktivem Caesium bzw. von radioaktiven Caesiumionen als auch von nichtradioaktivem Caesium bzw. von nichtradioaktiven Caesiumionen in den menschlichen oder tierischen Körper zu vermindern, das heißt in ihrer Konzentration zu reduzieren. Das Medizinprodukt oder andere pharmazeutische Produkt ist in Analogie zu den Lebewesen nicht in der Lage, zwischen den verschiedenen Isotopen des Caesiums zu unterscheiden. Das Klinoptilolith bindet das einfach positiv geladene Caesiumion, welches in Form seiner Salze auftritt, in ähnlicher Weise wie andere anorganische und organische Kationen und tauscht bei diesem Vorgang die in Klinoptilolith vorhandenen Calcium- bzw. Magnesiumionen gegen das Caesiumion aus. Ein derartiger Vorgang kann auch als Ionenaustauschreaktion bezeichnet werden. Der Ionenaustausch erfolgt üblicherweise, bis annähernd ein bestimmter Gleichgewichtszustand erreicht worden ist. Mit dem Stuhl kann das an das Klinoptilolith gebundene Caesium, bestehend aus dem harmlosen Caesium-133 und dem sehr gefährlichen Caesium-137, nach und nach den menschlichen Körper verlassen.

Gemäß einer ebenso vorteilhaften Ausführungsform ist das Medizinprodukt oder andere pharmazeutische Produkt mit dem modifizierten Klinoptilolith geeignet, bei vorbeugender und/oder rechtzeitiger Einnahme die Resorption von Caesium in Form seiner Verbindungen zu reduzieren. Des Weiteren ist das modifizierte Klinoptilolith vorzugsweise geeignet, bei Einnahme des Medizinproduktes oder anderen pharmazeutischen Produktes über einen bestimmten Zeitraum bereits im Lebewesen vorhandene und resorbierte Caesiumionen nach deren Einnahme schrittweise zu verringern, das heißt bezüglich ihrer Konzentration zu reduzieren. Durch eine nachgewiesen fehlende systemische Toxizität von "froximun toxaprevent® pure" kann man zum Beispiel dieses Medizinprodukt im Falle einer drohenden ungewollten Aufnahme von Caesium-137 vorbeugend einnehmen oder - sofern Caesium-137 bereits aufgenommen worden ist - zur Reduktion der Konzentration des im Körper möglicherweise bereits vorhandenen Caesium-137 einsetzen.

Die Caesium-bindende Eigenschaft von entsprechenden Präparaten bzw. Medizinprodukten wird im Detail dadurch sichtbar, dass man dem Futter von Versuchsmäusen einerseits Caesiumchlorid beimischt und andererseits Caesiumchlorid plus das Präparat für einen Untersuchungszeitraum dem Futter hinzufügt und schließlich beide Versuchsreihen miteinander vergleicht. Es zeigte sich zum Beispiel, dass, im Falle der Beimischung des Medizinproduktes "froximun toxaprevent® pure" plus Caesiumchlorid zum Futter, eine deutliche Reduktion der Caesiumaufnahme in den Körper der Labortiere erfolgte, im Vergleich zu jenen Tieren, die ausschließlich das Caesiumchlorid im Futter hatten. Darüber hinaus besitzt das Medizinprodukt "froximun toxaprevent® pure" auch die Eigenschaft, die Konzentration von bereits in Lebewesen vorhandenen Caesiumverbindungen bzw. Caesiumsalzen nach deren Einnahme über einen bestimmten Zeitraum hinweg schrittweise zu reduzieren. Im Falle einer Belastung durch Caesium-137 ergibt sich im Ernstfall durch Einnahme des Medizinproduktes eine für den Betroffenen verhältnismäßig einfach durchführbare Maßnahme der Dekontamination.

Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
- **Figur 1:**: eine grafische Darstellung der Ergebnisse einer Labortierstudie zu den Caesium-bindenden Eigenschaften eines klinoptilolithhaltigen Präparates nach oraler Aufnahme von Caesium-angereichertem Futter und
- **Figur 2:**: eine grafische Darstellung der Ergebnisse einer Labortierstudie zu den Caesium-bindenden Eigenschaften des klinoptilolithhaltigen Präparates bei gleichzeitiger oraler Aufnahme des Präparates und von Caesium.

Zunächst werden anhand von *In*-*vitro*-Versuchen die Caesium-bindenden Eigenschaften eines klinoptilolithhaltigen Präparates, in dem das Klinoptilolith durch Reinigung und Zerkleinerung modifiziert ist, untersucht. Es handelt sich bei diesem Präparat um das Medizinprodukt "froximun® toxaprevent pure". Das Medizinprodukt "froximun toxaprevent® pure" ist bereits zugelassen für die Bindung von Schwermetallen, wie Blei und Quecksilber, aber auch zur Bindung von Ammonium und von verschiedenen biogenen Aminen. In diesem Zusammenhang wird auf die Druckschriften DE 10 2005 053 090 A1 und WO 2010/057849 A1 verwiesen, die auf die Anmelderin der vorliegenden Erfindung zurückgehen. Die Partikel des modifizierten Klinoptiloliths weisen nach der Zerkleinerung Partikelgrößen von 2 bis 15 µm auf, vorzugsweise Partikelgrößen von 3 bis 10 µm. Die Partikel des modifizierten Klinoptiloliths weisen dabei Zetapotenziale von 20 bis 26 mV und eine spezifische Oberfläche von 30 bis 60 m²/g auf.

### Ausführungsbeispiel I:

Man öffnet eine Kapsel des Präparates "froximun toxaprevent® pure" und wiegt 50 mg Kapselinhalt in einem Erlenmeyer-Kolben ein. Man gibt 50 ml einer wässrigen Lösung zu, die Caesiumchlorid in einer Konzentration von 10 mg/l Caesium enthält. Man rührt die Lösung bei 37 °C für 17 Stunden und filtriert über einen Filter mit einer Porennennweite von 0,2 µm. Das Filtrat sollte danach klar sein. Die Analyse des Filtrates ergibt eine Konzentration für Caesium von 0,32 mg/l.

### Ausführungsbeispiel II:

Man öffnet eine Kapsel "froximun toxaprevent® pure" und wiegt 50 mg Kapselinhalt in einem Becher aus Polypropylen ein. Man gibt 12,5 ml einer wässrigen Lösung zu, die Caesiumchlorid in einer Konzentration von 40 mg/l Caesium enthält. Man rührt die Lösung bei Zimmertemperatur für 10 Stunden und filtriert über einen Filter mit einer Porennennweite von 0,2 µm. Das klare Filtrat ergibt eine Caesium-Konzentration von 0,18 mg/l.

### Ausführungsbeispiel III:

Man wiegt 500 mg "froximun toxaprevent® pure" in einem Erlenmeyer-Kolben mit Schliff ein und gibt 17 ml einer wässrigen Lösung zu, die Caesiumchlorid in einer Konzentration von 25 mg/l an Caesium enthält. Man rührt die Lösung bei Zimmertemperatur für 10 Stunden und filtriert anschließend über einen Filter. Für das klare Filtrat ergibt die Analyse eine einer Caesium-Bindung von 99,6 % entsprechende Caesium-Konzentration.

### Ausführungsbeispiel IV:

Man öffnet eine Kapsel "froximun toxaprevent® pure" und wiegt 50 mg Kapselinhalt in einem Erlenmeyer-Kolben ein. Man gibt 20,0 ml einer wässrigen Lösung zu, die Caesiumchlorid in einer Konzentration von 20 mg/l Caesium enthält. Man rührt die Lösung im Eiswasserbad für 20 Stunden und filtriert die Lösung über einen 0,2 µm-Filter. Das Filtrat ist danach klar. Die Caesium-Analyse ergibt eine Konzentration von 0,83 mg/l.

### Ausführungsbeispiel V:

Es wurden *In*-*vivo*-Versuche in Form von Fütterungsstudien an Mäusen mit "froximun toxaprevent® pure" durchgeführt. Die erste Fütterungsstudie erfolgte mit jeweils 3 Gruppen von je 20 weiblichen *Bälb*/*c*-Mäusen. Alle drei Gruppen wurden mindestens 10 Tage im 1. Zyklus gefüttert. Nach 10 Tagen wurde von jeder Gruppe die Hälfte der Tiere getötet, analysiert und ausgewertet. Für die verbleibenden Tiere begann ein zweiter Fütterzyklus für weitere 10 Tage. Danach wurden alle noch verbliebenen Tiere getötet, analysiert und ausgewertet.

Gruppe 1: Diese Gruppe bestand aus 20 Tieren für den Negativtest ohne CsCl im Futter. Diese Gruppe wurde durchgehend über den gesamten Zeitraum mit Normal-Futter ernährt. Nach 10 Tagen wurden die ersten 10 Tiere (Gruppe 1 a) getötet, analysiert und ausgewertet und nach 10 weiteren Tagen die verbleibenden 10 Tiere (Gruppe 1 b).

Gruppe 2: Diese Gruppe bestand aus 20 Tieren für den Positivtest mit Caesiumchlorid (CsCl) im ersten Fütterzyklus. Von dieser Gruppe wurden die ersten 10 Tiere (Gruppe 2a) nach 10 Tagen getötet, analysiert und ausgewertet. Die verbleibenden 10 Tiere (Gruppe 2b) dieser Gruppe wurden im zweiten Fütterzyklus nur mit Normal-Futter ernährt, also ohne CsCl. Nach weiteren 10 Tagen Fütterung wurden auch diese Tiere getötet, analysiert und der Gehalt an Caesium im Organismus ermittelt.

Gruppe 3: Diese Gruppe bestand aus 20 Tieren für den Test mit "froximun toxaprevent® pure". Die Fütterung fand in den ersten 10 Tagen parallel zur Gruppe 2 statt. Das Futter wurde also ebenfalls mit Caesiumchlorid (CsCl) angereichert und an alle Tiere dieser Gruppe verfüttert. Nach 10 Tagen wurden die ersten 10 Tiere (Gruppe 3a) dieser Gruppe getötet und die Konzentration von Caesium im Organismus parallel zur Gruppe 2 ermittelt.

Im zweiten Fütterzyklus wurde den verbleibenden 10 Tieren Normal-Futter mit "froximun toxaprevent® pure" verabreicht. Nach 10 Tagen Fütterung mit "froximun toxaprevent® pure" wurden diese Tiere (Gruppe 3b) getötet und ebenfalls analysiert.

Die Ergebnisse der Caesium-Konzentrationen wurden danach verglichen und die Konzentrationsunterschiede der 2. und 3. Fütterungsgruppe (Gruppen 2b und 3b) gegenübergestellt. Es wurde eine Reduzierung der Caesium-Konzentration im Organismus der letzten 10 Tiere der Gruppe 3 gegenüber den letzten 10 Tieren der Gruppe 2 ermittelt.

Die **Tabelle 1** enthält einen Überblick über das angewendete Fütterungsschema.

**Tabelle 1**

| | Gruppe 1 | Gruppe 2 | Gruppe 3 |
|---|---|---|---|
| 10 Tage (20 Tiere) | Normalfutter | Normalfutter +CsCl | Normalfutter+CsCl |
| 20 Tage (10 Tiere) | Normalfutter | Normalfutter | Normalfutter+ "froximun toxaprevent® pure" |

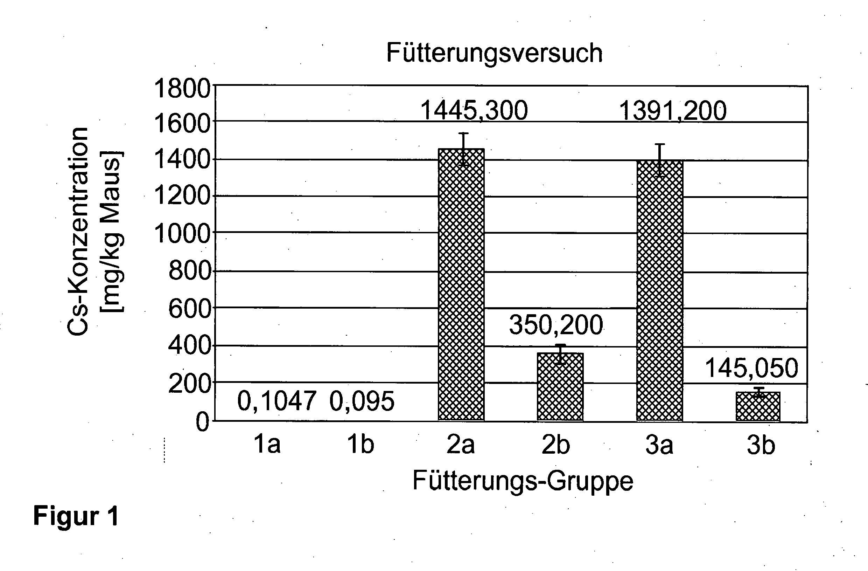

Die **Figur 1** zeigt eine grafische Darstellung der Ergebnisse der Labortierstudie zu den Caesium-bindenden Eigenschaften des Medizinproduktes "froximun® toxaprevent pure" nach oraler Aufnahme von Caesium-angereichertem Futter. Die Gruppe 1a erhielt 10 Tage lang Normalfutter ohne Zusätze. Der Wert der Caesium-Konzentration liegt annähernd bei 0 mg/kg Maus. Die Gruppe 1 b erhielt 20 Tage lang Normalfutter ohne Zusätze. Auch im Falle der Gruppe 1 b liegt die Caesium-Konzentration annähernd bei 0 mg/kg Maus.

Die Gruppe 2a erhielt 10 Tage lang Normalfutter, das mit Caesiumchlorid vermischt war. Die Caesiumkonzentration betrug nach diesen 10 Tagen 1445,3 mg/kg Maus. Die Gruppe 2b erhielt zunächst 10 Tage lang ebenfalls Normalfutter, das mit Caesiumchlorid vermischt war. Danach erhielt die Gruppe 2b bis zum 20. Tag Normalfutter ohne Caesium-Zusätze. Die Caesium-Konzentration lag nach dem 20. Tag bei 350,2 mg/kg Maus.

Die Gruppe 3a erhielt 10 Tage lang Normalfutter, das mit Caesiumchlorid vermischt war. Die Caesiumkonzentration betrug nach diesen 10 Tagen 1391,2 mg/kg Maus. Die Gruppe 3b erhielt zunächst 10 Tage lang ebenfalls Normalfutter, das mit Caesiumchlorid vermischt war. Danach erhielt die Gruppe 3b bis zum 20. Tag Normalfutter ohne Caesium-Zusätze, aber mit Zusätzen an "froximun® toxaprevent pure". Der Caesiumgehalt betrug nach 20 Tagen 145,05 mg/kg Maus.

### Bewertung der Testergebnisse:

Im vorliegenden Versuch wurden die Caesium-bindenden Eigenschaften des Produktes "froximun® toxaprevent pure" nach oraler Aufnahme von Caesiumhaltigem Futter untersucht. Aus **Figur 1** ist zu erkennen, dass unter den gegebenen Bedingungen der vorliegenden Studie der Caesiumgehalt der Tiere, die mit Caesium-haltigem Futter über einen Zeitraum von 10 Tagen gefüttert wurden, im Organismus bei etwa 1400 mg/kg Körpergewicht (1,4 mg/g Körpergewicht) liegt. Bei den Tieren, die anschließend mit Normalfutter gefüttert wurden, lag der Caesiumgehalt bei durchschnittlich 350 mg/kg Körpergewicht. Bei den Tieren, die anschließend mit "froximun® toxaprevent pure" gefüttert wurden, lag der Caesiumgehalt bei durchschnittlich 145 mg/kg Körpergewicht. Daraus lässt sich schließen, dass im Organismus befindliches Caesium mit Aufnahme des Präparates "froximun® toxaprevent pure" schneller eliminiert wird. Unter den im vorliegenden Versuch beschriebenen Bedingungen entspricht die Differenz zwischen den mit Normalfutter gefütterten Tieren und den mit "froximun® toxaprevent pure" gefütterten Tieren einer beschleunigten Caesium-Eliminierung aus dem Organismus von 58,6 %.

Anhand einer zweiten Labortierstudie wurden die Caesium-bindenden Eigenschaften des Medizinproduktes "froximun® toxaprevent pure" bei gleichzeitiger Aufnahme des Medizinproduktes und Caesium mit dem Futter untersucht. Analysiert wurde die Caesiumkonzentration im Körper nach einwöchiger Fütterung mittels Massenspektrometrie mit induktiv gekoppeltem Plasma (ICP-MS). Für die Fütterungsstudie wurden 3 Gruppen von je 10 weiblichen *Balb*/*c*-Mäusen herangezogen.

Da aus den *In*-*vitro*-Versuchen bekannt ist, dass das Medizinprodukt Caesium binden kann, wurde ein Futter hergestellt, das in diesem Verhältnis Caesium und Medizinprodukt enthält.

Es wurde Caesiumchlorid als Caesiumquelle verwendet:
0,5 mg Cs (M = 132,91 g/mol) → 500 mg Medizinprodukt,
1,0 mg Cs → 1 g Medizinprodukt,
1,27 mg CsCl (M = 168,36 g/mol) → 1 g Medizinprodukt.

Als Positivkontrolle diente Futter, welches nur Caesiumchlorid (CsCl) enthielt. Als Negativkontrolle wurde Futter ohne Caesiumchlorid und ohne Medizinprodukt hergestellt.

Die **Tabelle 2** enthält einen Überblick über die Futterherstellung.

**Tabelle 2:**

| | |
|---|---|
| Normalfutter | 291 g Futtermehl + 9 g Gelatine + H₂O |
| CsCI-Futter | 291 g Futtermehl + 9 g Gelatine + 38,1 mg CsCl + H₂O |
| Produkt + CsCI-Futter | 261 g Futtermehl + 9 g Gelatine + 30 g Produkt + 38,1 mg CsCl + H₂O |

Fütterung: Die Futter- und Wasseraufnahme erfolgte *ad libitum* über den Zeitraum von 7 Tagen. Die Tiergewichte und die Futtermengen wurden vor Versuchsbeginn und nach Versuchsende bestimmt.

Tierpräparation: Nach Abschluss der Fütterung wurden die Mäuse durch cervicale Dislokation getötet. Magen und Darm wurden entfernt, damit dort befindliche Futterreste nicht die quantitative Caesiumbestimmung im Körpergewebe beeinflussen. Die Tiere wurden einzeln komplett, bis auf die Organe Fell, Zehen und Schwanz, homogenisiert und anschließend lyophilisiert, das heißt gefriergetrocknet.

Caesiumbestimmung: Nach Druckaufschluss wurden die Lyophilisate mittels ICP-MS auf ihren Caesiumgehalt untersucht. Die analytische Bestimmungsgrenze der Methode liegt bei 0,0025 mg/kg.

Die **Tabelle 3** gibt einen Überblick über die verschiedenen Zusammensetzungen des jeweils verabreichten Futters und über den Futterverbrauch.

**Tabelle 3**

| | Futterverbrauch [g/10 Tiere/7 Tage] | Caesiumgehalt [mg/kg] |
|---|---|---|
| Normalfutter | 196,8 | 0,23 |
| CsCI-Futter | 199,2 | 95,5 |
| Medizinprodukt + Caesiumchlorid-Futter | 208,0 | 97,7 |

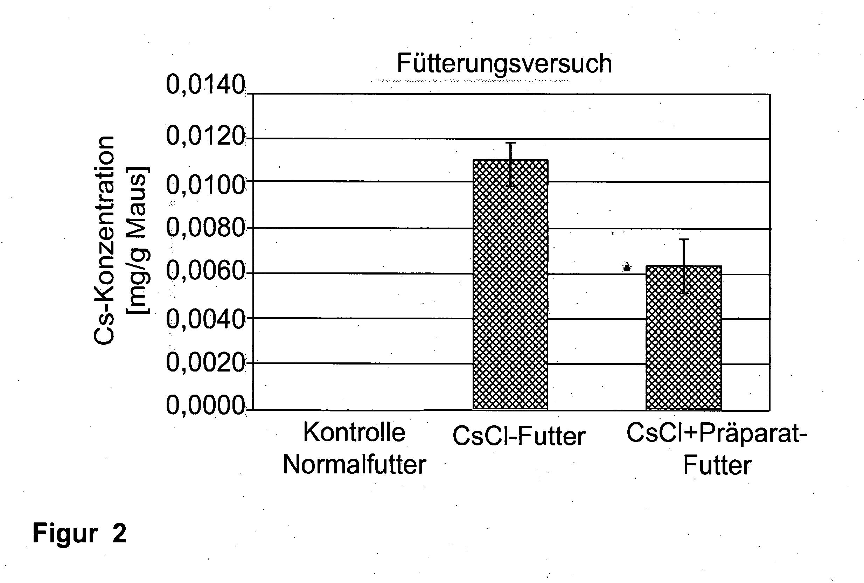

Die **Figur 2** zeigt eine grafische Darstellung der Ergebnisse der Labortierstudie zu den Caesium-bindenden Eigenschaften des Medizinproduktes "froximun® toxaprevent pure" bei gleichzeitiger oraler Aufnahme des Medizinproduktes und von Caesium.

Aus **Figur 2** ist zu erkennen, dass unter den gegebenen Bedingungen der vorliegenden Studie bei gleichzeitiger oraler Applikation von Caesium und dem Medizinprodukt die Aufnahme von Caesium in das Körpergewebe der Labortiere im Durchschnitt 42 % geringer ist als bei den Tieren, die Caesiumhaltiges Futter ohne das Medizinprodukt "froximun® toxaprevent pure" bekommen haben.

## Patentansprüche

1. Klinoptilolith oder klinoptilolithhaltigen Gemischen zur Verwendung als Medizinprodukt oder eines anderen pharmazeutischen Produktes, zur Aufnahme und Ausleitung von Caesiumionen aus dem menschlichen oder tierischen Körper, wobei das Klinoptilolith durch Reinigung und Zerkleinerung modifiziert ist, **dadurch gekennzeichnet, dass** die Partikel des modifizierten Klinoptiloliths nach der Zerkleinerung Partikelgrößen von 2 bis 15 µm aufweisen.

2. Klinoptilolith oder klinoptilolithhaltigen Gemische zur Verwendung nach Anspruch 1, **dadurch gekennzeichnet, dass** die Partikel des modifizierten Klinoptiloliths nach der Zerkleinerung Partikelgrößen von 3 bis 10 µm aufweisen.

3. Klinoptilolith oder klinoptilolithhaltigen Gemischen zur Verwendung nach Anspruch 1 oder 2, **dadurch gekennzeichnet, dass** die Partikel des modifizierten Klinoptiloliths Zetapotenziale von 20 bis 26 mV aufweisen.

4. Klinoptilolith oder klinoptilolithhaltigen Gemischen zur Verwendung nach einem der Ansprüche 1 bis 3, **dadurch gekennzeichnet, dass** die Partikel des modifizierten Klinoptiloliths eine spezifische Oberfläche von 30 bis 60 m²/g aufweisen.

## Claims

1. Klinoptilolith or klinoptilolith-containing mixtures for use as a medicinal product or as another pharmaceutical product, for the absorption and removal of caesium ions from the human or animal body, wherein the klinoptilolith is modified by purification and comminution, **characterized in that** the particles of the modified klinoptilolith after comminution have particle sizes of 2 to 15 µm.

2. Klinoptilolith or klinoptilolith-containing mixtures for use according to claim 1, **characterized in that** the particles of the modified klinoptilolith after comminution have particle sizes of 3 to 10 µm.

3. Klinoptilolith or klinoptilolith-containing mixtures for use according to claim 1 or 2, **characterized in that** the particles of the modified klinoptilolith have zeta potentials of 20 to 26 mV.

4. Klinoptilolith or klinoptilolith-containing mixtures for use according to any of claims 1 to 3, **characterized in that** the particles of the modified klinoptilolith have a specific surface of 30 to 60 m²/g.

## Revendications

1. Clinoptilolithe ou de mélanges contenant de la clinoptilolithe pour l'utilisation en tant que produit médicamenteux ou autre produit pharmaceutique, destinés à l'absorption ou à l'évacuation d'ions de césium du corps humain ou animal, la clinoptilolithe étant modifiée par nettoyage ou fragmentation, **caractérisée en ce que** les particules de la clinoptilolithe modifiée après la fragmentation présentent des tailles de particules de 2 à 15 µm.

2. Clinoptilolithe ou de mélanges contenant de la clinoptilolithe pour l'utilisation selon la revendication 1, **caractérisée en ce que** les particules de la clinoptilolithe modifiée après la fragmentation présentent des tailles de particules de 3 à 10 µm.

3. Clinoptilolithe ou de mélanges contenant de la clinoptilolithe pour l'utilisation selon l'une quelconque des revendications 1 ou 2, **caractérisée en ce que** les particules de la clinoptilolithe modifiée présentent des potentiels zêta de 20 à 26 mV.

4. Clinoptilolithe ou de mélanges contenant de la clinoptilolithe pour l'utilisation selon l'une quelconque des revendications 1 à 3, **caractérisée en ce que** les particules de la clinoptilolithe modifiée présentent une surface spécifique de 30 à 60 m²/g.
